# EUROPEAN PATENT APPLICATION

(11) **EP 4 011 287 A1**
(43) Date of publication of application: **15.06.2022**
(21) Application number: 20213341.9
(22) Date of filing: 11.12.2020
(51) Int. Cl.: A61B 5/024, A61N 1/39

(54) **CARDIOPULMONARY RESUSCITATION DECISION SUPPORT**

(71) Applicant: Koninklijke Philips N.V., 5656 AG Eindhoven (NL)
(72) Inventor: MUEHLSTEFF, Jens, 5656 AE Eindhoven (NL); WIJSHOFF, Ralph Wilhelm Christianus Gemma Rosa, 5656 AE Eindhoven (NL); VAN DE LAAR, Jakob, 5656 AE Eindhoven (NL); VEENSTRA, Hugo, 5656 AE Eindhoven (NL); JANSSEN, Anthonius Petrus Gerardus Emanuel, 5656 AE Eindhoven (NL)
(74) Representative: Philips Intellectual Property & Standards

(57) **Abstract**

According to an aspect, there is provided a system for providing cardiopulmonary resuscitation decision support. The system comprises: a photoplethysmography sensing unit configured to determine one or more PPG signals; a communication unit configured to receive, from at least one of an external device and a motion sensing unit, a signal corresponding to chest compressions during compression therapy; a processing unit configured to: determine presence or absence of a spontaneous pulse based on the received signal corresponding to chest compressions and the one or more PPG signals; and control the communication unit to send an instruction to the external device or a user interface of the system based on the presence or absence of a spontaneous pulse.

## Description

### FIELD OF THE INVENTION

The present disclosure relates to a system for providing cardiopulmonary resuscitation (CPR) decision support and a method for operating thereof.

### BACKGROUND OF THE INVENTION

During cardiopulmonary resuscitation (CPR), pulse checks are mainly performed by manual palpation. Palpation requires interruption of the chest compressions, and is a subjective, challenging, and time-consuming procedure. As a result, manual palpation can interrupt the chest compressions for longer than the 10 seconds duration as recommended by medical guidelines. Palpations tend to be lengthy and time-consuming, especially in the case of potentially perfusing electrocardiography (ECG) rhythms. These long interruptions can negatively impact the outcome of the CPR.

There are some currently available objective methods for checking the status of the circulation during CPR. For example, via invasive arterial blood pressure (iABP) measurements, one can directly see when the heart resumes beating and obtain a quantified measure of the status of the circulation and also during compressions. However, iABP measurements are invasive and therefore they are not common practice during CPR. Alternatively, waveform capnography is a less invasive objective method that provides information about the status of the circulation. Nevertheless, capnography still requires intubation, the interpretation of the end-tidal CO2 level requires well-controlled ventilations and it does not provide any measure of the spontaneous pulse rate (PR).

### SUMMARY OF THE INVENTION

As noted above, there are a number of disadvantages associated with currently available methods for checking the status of the circulation during compression therapy. Furthermore, the pulse oximetry socket of some currently available systems does not support intended application scenarios of CPR, as such applications would require an internal signal fusion along with significant changes to the firmware in the monitors. Also, the measurement sockets at currently available monitors do not facilitate access for additional sensor signals (e.g. accelerometer, environmental light conditions, etc.) with the required synchronicity in signal acquisition. To date, there is no method for supporting pulse detection during CPR which is objective, non-invasive, and easy-to-use, and which offers flexibility in the use of reference signals corresponding to compressions.

In the present disclosure, motion-robust photoplethysmography (PPG) is employed for the purpose of pulse determination during compression therapy. Preclinical and clinical data have demonstrated good potential of using PPG for detecting a spontaneous pulse during pauses and ongoing compressions during CPR. Furthermore, algorithms have been developed for automated detection of a spontaneous pulse in a PPG signal during CPR. For example, international patent application WO 2017/211814 describes a technique by which a PPG signal can be used to detect a return (or continued presence) of a spontaneous pulse during the application of CPR. These algorithms rely on frequency analysis and require a reference signal to handle artefacts caused by compressions so as to reveal the presence of a potentially existing spontaneous pulse signal. Currently, the trans-thoracic impedance signal from a defibrillator or a monitor is used as a reference signal for the chest compressions. However, the need for a full integration of such algorithms in a monitor (or a defibrillator) limits the applicability of the approach.

Nevertheless, one of the ways to realize PPG as a way to detect a spontaneous pulse during compression therapy is to implement essential system components external of the monitor or the defibrillator so as to facilitate basic functionalities. This concept is sometimes referred to as "Smart Cables" or "Smart Measurements". In some currently available systems, measurement hardware is integrated into cables that are connected by a standardized interface. In these systems, the monitor could be the user interface, but it could also be the processing unit (in addition).

It would therefore be advantageous to provide an improved system for enabling CPR decision support based on motion-robust low-perfusion pulse oximetry as a monitoring tool that can be used in combination with an external patient monitor, a defibrillator, or a CPR robot, etc., and that supports both manual CPR and automated CPR.

According to a first specific aspect, there is provided a system for providing cardiopulmonary resuscitation (CPR) decision support, the system comprising: a photoplethysmography, PPG, sensing unit configured to determine one or more PPG signals at a measurement site on a subject; a communication unit configured to receive, from at least one of an external device and a motion sensing unit, a signal corresponding to chest compressions during compression therapy on the subject, wherein the external device is one of: an external patient monitor, a defibrillator, and a CPR robot; a processing unit configured to: determine presence or absence of a spontaneous pulse based on the received signal corresponding to chest compressions during compression therapy on the subject and the one or more PPG signals; and control the communication unit to send an instruction to the external device or a user interface of the system, based on the determined presence or absence of a spontaneous pulse, wherein the instruction indicates at least one of: to output, via a user interface of the external device or a user interface of the system, at least one of the determined presence or absence of a spontaneous pulse; advice on further checking for pulse presence and/or withholding vasopressors to the subject; to stop compression therapy on the subject or to provide a prompt for stopping compression therapy; and to adjust a compression rate of compression therapy on the subject or to provide a prompt for adjusting the compression rate of compression therapy.

In some embodiments, the communication unit may be configured to be connected to the external device wirelessly or via a wired connection.

In some embodiments, the signal received from the external device may be associated with at least one of: an impedance measurement value, a compression depth measurement value, a compression velocity measurement value, a compression acceleration measurement value, a compression force measurement value, a robot compression signal.

In some embodiments, the communication unit may be further configured to receive a synchronization signal from the external device, and the processing unit may be further configured to perform synchronization of the signal corresponding to chest compressions with the one or more PPG signals based on the synchronization signal, and to determine presence or absence of a spontaneous pulse based on the synchronized signals.

In some embodiments, the instruction indicating to stop compression therapy on the subject may comprise an instruction to stop compression therapy on a regular basis for a predetermined time interval, when absence of a spontaneous pulse is determined during compression therapy on the subject.

In some embodiments, the processing unit may be further configured to determine whether there is an uncertain and/or weak spontaneous pulse based on the received signal corresponding to chest compressions during compression therapy on the subject and the one or more PPG signals. In these embodiments, the instruction indicating to stop compression therapy on the subject may comprise an instruction to stop compression therapy on a regular basis for a predetermined time interval, when it is determined that there is an uncertain and/or weak spontaneous pulse during compression therapy on the subject.

In some embodiments, the PPG sensing unit may comprise at least one of: a nasal alar PPG sensor, a nasal columella PPG sensor, an ear concha PPG sensor, and a forehead PPG sensor.

In some embodiments, the PPG sensing unit may comprise more than one of: a nasal alar PPG sensor, a nasal columella PPG sensor, an ear concha PPG sensor, and a forehead PPG sensor, and the user interface may be configured to receive a user input indicating at least one sensor to activate for determining the one or more PPG signals. Alternatively or in addition, the processing unit may receive signal quality indicator(s) corresponding to one or more of the PPG sensors, and one or more PPG sensors may be activated based on the received signal quality indicator(s). Alternatively or in addition, one or more PPG sensors may be activated based on the received user input and the received signal quality indicator(s).

In some embodiments, the processing unit may be further configured to: acquire a core body temperature value of the subject; and control the communication unit to send an instruction indicating to output, via a user interface of the external device or a user interface of the system, at least one of: the detected core body temperature, an indication of whether the detected core body temperature is within a predetermined target range or at a predetermined target value, and an indication of whether the detected core body temperature is approaching a predetermined target range or a predetermined target value.

In some embodiments, the processing unit may be further configured to acquire an electrocardiography, ECG, signal associated with the subject, and wherein determining presence or absence of a spontaneous pulse is further based on the acquired ECG signal.

In some embodiments, the processing unit may be further configured to acquire a signal indicating nasal flow of the subject, wherein controlling the communication unit to send an instruction to the external device or a user interface of the system is further based on the acquired signal indicating nasal flow of the subject.

In some embodiments, the processing unit may be further configured to: determine whether amplitudes and/or a signal quality corresponding to the detected motions correlated to chest compressions during compression therapy on the subject are within a predetermined target range; and control the communication unit to send an instruction to output, via a user interface of the external device or a user interface of the system, at least one of: an indication of the result of the determination of whether the amplitudes and/or the signal quality are within a predetermined target range, and an instruction to improve the amplitude and/or signal quality.

In some embodiments, the processing unit may be implemented as part of the external device, and the processing unit is configured to connect to the communication unit and the PPG sensing unit wirelessly.

According to a second specific aspect, there is provided a method for operating a system for providing cardiopulmonary resuscitation (CPR) decision support, wherein the system comprises a photoplethysmography (PPG) sensing unit, a communication unit, and a processing unit, the method comprising: determining, at the PPG sensing unit, one or more PPG signals at a measurement site on a subject; receiving, at the communication unit, a signal corresponding to chest compressions during compression therapy on the subject from at least one of an external device and a motion sensing unit, wherein the external device is one of: an external patient monitor, a defibrillator, and a CPR robot; determining, at the processing unit, presence or absence of a spontaneous pulse based on the received signal corresponding to chest compressions during compression therapy on the subject and the one or more PPG signals; and controlling, at the processing unit, the communication unit to send an instruction to the external device or a user interface of the system, based on the determined presence or absence of a spontaneous pulse, wherein the instruction indicates at least one of: to output, via a user interface of the external device or a user interface of the system, at least one of the determined presence or absence of a spontaneous pulse ; advice on further checking for pulse presence and/or withholding vasopressors to the subject; to stop compression therapy on the subject or to provide a prompt for stopping compression therapy; and to adjust a compression rate of compression therapy on the subject or to provide a prompt for adjusting the compression rate of compression therapy.

According to aspects and embodiments as described above, the limitations of existing techniques are addressed. In particular, at least some of the above-described aspects and embodiments provide a CPR decision support solution that is cost-effective compared to a fully integrated PPG measurement approach in a monitor or a defibrillator. Some aspects and embodiments also offer easy integration and/or implementation with third party systems and support closed-loop CPR therapy using CPR robots (e.g. in the context of a CPR robot, compressions would not be interrupted if no pulse is present, and compressions can be automatically stopped according to an instruction when pulse presence is detected). Moreover, some aspects and embodiments allow the required measurements for CPR-proof pulse oximetry to be used as reference for the detection of a spontaneous pulse, while offering flexibility in the use of various different signals corresponding to compressions. In addition, since at least some of the aspects and embodiments adopt both wired and wireless transmission techniques and modes, clutter issues in particular during cardiopulmonary resuscitation can be reduced or avoided.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of a system for providing cardiopulmonary resuscitation (CPR) decision support, according to an embodiment; and
Fig. 2 is a flow chart for a method for operating a system for providing cardiopulmonary resuscitation (CPR) decision support, according to an embodiment.

### DETAILED DESCRIPTION OF EMBODIMENTS

As noted above, there is provided an improved system and a method of operating the same, which address the existing problems.

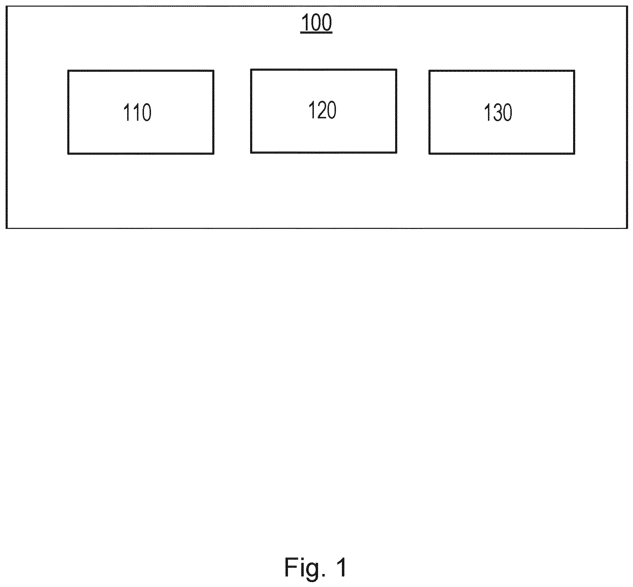

Fig. 1 is a block diagram of a system 100 for providing cardiopulmonary resuscitation (CPR) decision support. As will be explained in more detail below, the system 100 can be used in conjunction with an external patient monitor, a defibrillator (e.g. an automatic defibrillator or a hospital defibrillator), or a CPR robot. Thus, the system 100 does not require full integration into a patient monitor or a defibrillator in order to perform the functions as described below, and it can be used in scenarios such as out-of-hospital CPR.

As shown in Fig. 1, the system 100 comprises a photoplethysmography (PPG) sensing unit 110, a communication unit 120, and a processing unit 130. The connections between the components of the system 100, particularly between the communication unit 120 and the other components (including external entities) and between the processing unit 130 and the other components, can be implemented wirelessly, e.g. Global System for Mobile Communications (GSM), Bluetooth, Bluetooth Low Energy, and/or Near-field Communication (NFC). Furthermore, in some embodiments at least some connections between the components of the system 100 can be switchable between wired and wireless.

The PPG sensing unit 110 is configured to determine one or more PPG signals at a measurement site on a subject. The PPG sensing unit 110 may comprise a low-perfusion oximetry sensor, and more specifically the PPG sensing unit 110 may comprise at least one of: a nasal alar PPG sensor, a nasal columella PPG sensor, an ear concha PPG sensor, and a forehead PPG sensor.

The communication unit 120 is configured to receive, from at least one of an external device and a motion sensing unit, a signal corresponding to chest compressions during compression therapy on the subject. The external device is one of: an external patient monitor, a defibrillator, and a CPR robot. The signal received from the external device may be associated with at least one of: an impedance measurement value, a compression depth measurement value, a compression velocity measurement value, a compression acceleration measurement value, a compression force measurement value, and a robot compression signal.

In some embodiments, the motion sensing unit may be a part of the system 100 (although the motion sensing unit is not shown in Fig. 1). In this case, the motion sensing unit may be configured to detect motions correlated to chest compressions during compression therapy on the subject, and it may comprise one or more accelerometers. Alternatively or in addition, the motion sensing unit may be external to the system 100 (e.g. a motion sensing unit implemented in an external patient monitor).

In some embodiments, the communication unit 120 may be configured to be connected to the external device wirelessly or via a wired connection.

The processing unit 130 is configured to determine presence or absence of a spontaneous pulse based on the received signal corresponding to chest compressions during compression therapy on the subject (from the motion sensing unit and/or the external device), and the one or more PPG signals (from the PPG sensing unit 110). For example, accelerometry traces of the motions detected by a motion sensing unit (as represented by the signal from the motion sensing unit) can be used as a reference of chest compressions, such that the effects of compression therapy can be taken into account in determining presence or absence of a spontaneous pulse. In some embodiments, this determination may be performed according to one or more algorithms developed for the purpose of detecting a return (or continued presence) of a spontaneous pulse during the application of CPR, such as the technique described in international patent application WO 2017 /211814, which includes a method that results in the output of a result of a classified pulse condition ("no pulse, "pulse", and "indeterminate"), the detection being based on compression reference signals and PPG signals.

The processing unit 130 is further configured to control the communication unit 120 to send an instruction to the external device based on the determined presence or absence of a spontaneous pulse. The term "instruction" in the context of the present disclosure may refer to a signal which indicates to the external device to perform the relevant operation(s). In more detail, the instruction sent by the processing unit 130 may indicate at least one of: to output, via a user interface (e.g. a display screen) of the external device or a user interface of the system 100, at least one of: the determined presence or absence of a spontaneous pulse , advice on further checking for pulse presence and/or withholding vasopressors to the subject, to stop compression therapy on the subject or to provide a prompt for stopping compression therapy, and to adjust a compression rate of compression therapy on the subject or to provide a prompt for adjusting the compression rate of compression therapy. For example, a prompt for stopping compression therapy may include a user to confirm via user input at the external device whether compression therapy is to be stopped.

Therefore, upon receiving such instructions, the external device may perform the corresponding operation(s). For example, upon receiving an instruction from the processing unit 130 of the system 100, a CPR robot may be controlled to stop performing compression therapy on the subject.

More specifically, in some embodiments, the instruction indicating to stop compression therapy on the subject may comprise an instruction to stop compression therapy on a regular basis for a predetermined time interval (e.g. 5 seconds), when absence of a spontaneous pulse is determined during compression therapy on the subject. This may allow time for determining presence/absence of a spontaneous pulse in one or more compression-free PPG signals, as compared to longer pauses of unpredictable duration in case of manual CPR. This may in turn allow more reliable pulse detection, while still maintaining a high ratio of compression to compression-free time durations. Also, in some embodiments, the instruction to stop compression therapy may cause the external device to perform an automatic termination of the compression therapy upon receiving such instruction.

Also, in some embodiments, the instruction indicating to adjust a compression rate of compression therapy (or to provide a prompt for adjusting the compression rate of compression therapy) may be based on medical/protocol guidelines. This may allow a spontaneous pulse rate close to the compression rate to be tracked more easily.

Since the instruction to be sent to the external device is based on presence or absence of a spontaneous pulse, the system 100 enables patient safety to be improved especially when using automated CPR techniques (e.g. CPR robot), because instructions such as "stop compression therapy" can be provided which can cause the CPR robot to automatically stop compression therapy without requiring further human intervention. Moreover, the determined instruction enables patient care outcome to be improved, since unnecessary pulse checks and the associated interruptions in compression therapy can be avoided.

As mentioned above, the instruction may indicate to the external device or a user interface of the system 100 to output at least one of the determined presence or absence of a spontaneous pulse and advice on further checking for pulse presence and/or withholding vasopressors to the subject - this may be performed via a display screen of the user interface of the external device or a user interface of the system 100, for example. As a more specific example, the output may involve outputting, via the user interface, text information which reads "presence of a spontaneous pulse detected" or "presence of a spontaneous pulse not detected". Alternatively, this may be output via a light source by way of different colors (e.g. green indicating spontaneous pulse detected and red indicating spontaneous pulse not detected).

In some embodiments, the processing unit 130 may be implemented as part of the external device (and in these embodiments the external device may be part of the system, i.e. no longer an "external" device and can be referred as a "monitoring device" or "monitoring unit" of the system 100, or at least part(s) of it may be regarded as part of the system 100). In these embodiments, the processing unit 130 may be configured to connect to the communication unit 120 and the PPG sensing unit 110 wirelessly.

In some embodiments, the communication unit 120 may be further configured to receive a synchronization signal from the external device. In these embodiments, the processing unit 130 may be further configured to perform synchronization of the signal corresponding to chest compressions with the one or more PPG signals based on the synchronization signal, and to determine presence or absence of a spontaneous pulse further based on the synchronized signals.

In some embodiments, the processing unit 130 may be further configured to determine whether there is an uncertain and/or weak spontaneous pulse based on the received signal corresponding to chest compressions during compression therapy on the subject and the one or more PPG signals. In these embodiments, the instruction indicating to stop compression therapy on the subject may comprise an instruction to stop compression therapy on a regular basis for a predetermined time interval, when it is determined that there is an uncertain and/or weak spontaneous pulse during compression therapy on the subject.

As mentioned above, the PPG sensing unit 110 may comprise at least one of: a nasal alar PPG sensor, a nasal columella PPG sensor, an ear concha PPG sensor, and a forehead PPG sensor. In these embodiments, the processing unit 130 may be further configured to receive a user input indicating at least one sensor to activate for determining the one or more PPG signals. The processing unit 130 may then control the respective sensor(s) so as to perform relevant measurements for determining the one or more PPG signals. Alternatively or in addition, the processing unit 130 may receive signal quality indicator(s) corresponding to one or more of the PPG sensors, and one or more PPG sensors may be activated based on the received signal quality indicator(s). Alternatively or in addition, one or more PPG sensors may be activated based on the received user input and the received signal quality indicator(s). Therefore, user(s) can decide which sensor(s) to use based on, for example, best compatibility with the user's workflow. For example, this decision may be based on whether ventilation of the subject is performed using a mask or via intubation.

In some embodiments, one or more other key basic vital signs (e.g. Sp02, pulse rate, core body temperature, and respiration rate) can be monitored by the system 100 to enable determination of a return of spontaneous circulation (ROSC) or to provide further information on the patient condition after having achieved ROSC.

For example, in some embodiments, the processing unit 130 may be further configured to acquire a core body temperature (CBT) value of the subject, and to control the communication unit 120 to send an instruction to the external device or a user interface of the system 100 indicating to output, via a user interface of the external device or a user interface of the system 100, at least one of: the detected core body temperature, an indication of whether the detected core body temperature is within a predetermined target range or at a predetermined target value, and an indication of whether the detected core body temperature is approaching a predetermined target range or a predetermined target value. Since during CPR a controlled decrease and/or maintenance of appropriate CBT is important to the outcome of CPR (e.g. so as to preserve brain function), by indicating the CBT (and/or whether it is within a target range or at a target value) via a user interface of the external device or the system 100, user(s) can be provided with useful information during CPR which can help achieve a desired outcome more efficiently and effectively.

For this purpose, the system 100 may further comprise a core body temperature (CBT) sensing unit, the CBT sensing unit being configured to detect the core body temperature of the subject. Accordingly, in these embodiments, the CBT value of the subject may be acquired by the processing unit 130 from the CBT sensing unit. Alternatively, CBT value of the subject may be provided by a CBT sensing unit that is external to the system 100, e.g. a CBT sensing unit of the patient monitor.

In some embodiments, the processing unit 130 may be further configured to acquire an electrocardiography (ECG) signal associated with the subject. In these embodiments, the processing unit 130 may be configured to determine presence or absence of a spontaneous pulse further based on the acquired ECG signal. Specifically, in some embodiments the ECG signal may be used by the processing unit 130 to confirm organized electrical activity of the heart of the subject, which is a prerequisite for pulse presence.

For this purpose, the system 100 may further comprise an ECG sensing unit which is configured to detect an ECG signal associated with the subject. Accordingly, in these embodiments, the ECG signal may be acquired by the processing unit 130 from the ECG sensing unit. The ECG sensing unit may comprise, for example, ECG electrodes to be placed at skin contact measurement sites (e.g. the nose) on the subject. Alternatively, the ECG signal may be provided by an ECG sensing unit that is external to the system 100, e.g. from an ECG sensing unit of the patient monitor.

In some embodiments, the processing unit 130 may be further configured to acquire a signal indicating nasal flow of the subject. In these embodiments, the processing unit 130 may be configured to control the communication unit to send an instruction to the external device or a user interface of the system 100 further based on the acquired signal indicating nasal flow of the subject. For example, an instruction (e.g. such as whether and/or when to start or restart compression therapy, or follow-up action(s) after return of spontaneous circulation (ROSC)) can be provided based on a respiration rate of the subject that is derived, at the processing unit 130 or otherwise, from the signal indicating nasal flow of the subject. In some embodiments the processing unit 130 may be configured to determine presence or absence of a spontaneous pulse further based on the acquired signal indicating nasal flow of the subject, and thereby an instruction can be provided based on the presence or the absence of a spontaneous pulse.

For this purpose, the system 100 may further comprise a nasal flow sensing unit configured to detect a signal indicating nasal flow of the subject. Accordingly, in these embodiments, the signal indicating nasal flow of the subject may be acquired by the processing unit 130 from the nasal flow sensing unit. Alternatively, the signal indicating nasal flow of the subject may be provided by a nasal flow sensing unit that is external to the system 100, e.g. from a nasal flow sensing unit of the patient monitor.

In some embodiments, the processing unit 130 may be further configured to: determine whether amplitudes and/or a signal quality corresponding to the motions correlated to chest compressions during compression therapy on the subject (which may be extracted from signal(s) corresponding to chest compressions) are within a predetermined target range, and control the communication unit 120 to send an instruction to output, via a user interface of the external device or a user interface of the system 100, at least one of: an indication of the result of the determination of whether the amplitudes and/or the signal quality are within a predetermined target range (e.g. a warning that the signal quality is not sufficient enough to allow compression-induced oscillations in the detected motions to be discerned, or not sufficient enough for respective algorithm(s) for the purpose of spontaneous pulse presence detection), and an instruction to improve the amplitude and/or signal quality (e.g. to adjust a position of the motion sensing unit in a way such that sufficient and/or accurate motion sensing corresponding to compression-induced movements can be achieved).

Although not shown in the drawing, in some embodiments the system 100 may further comprise a user interface. The user interface may be for use in providing a user of the system 100 with information resulting from the techniques described herein. Alternatively or in addition, the user interface may be configured to receive a user input. For example, the user interface may allow a user of the system 100 to manually enter instructions, data, or information.

The user interface may be any user interface that enables the rendering (or output or display) of information to a user of the system 100. Alternatively or in addition, the user interface may be any user interface that enables a user of the system 100 to provide a user input, interact with and/or control the system 100. For example, the user interface may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

Although not shown in the drawing, in some embodiments the system 100 may comprise a communications interface (or circuitry) for enabling the system 100 to communicate with any interfaces, memories and/or devices that are internal or external to the system 100. The communications interface may communicate with any interfaces, memories and/or devices wirelessly or via a wired connection. For example, the communications interface may communicate with one or more user interfaces wirelessly or via a wired connection. Similarly, the communications interface may communicate with the one or more memories wirelessly or via a wired connection.

It will be appreciated that Fig. 1 only shows the components required to illustrate an aspect of the system 100 and, in a practical implementation, the system 100 may comprise alternative or additional components to those shown. For example, in some embodiments the system 100 may comprise a power source.

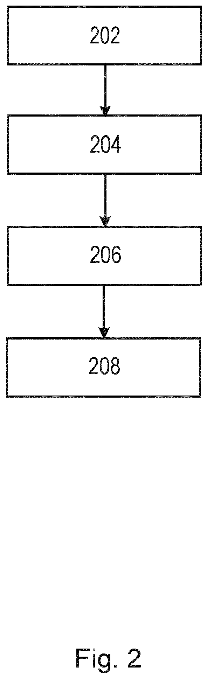

Fig. 2 is a flow chart for a method for operating a system for providing cardiopulmonary resuscitation (CPR) decision support, such as the system 100 as illustrated in Fig. 1. The system comprises a photoplethysmography (PPG) sensing unit, a communication unit, and a processing unit. In order to facilitate understanding, some of the description below will be made with reference to the various components of the system 100 as shown in Fig. 1.

With reference to Fig. 2, at block 202, one or more PPG signals at a measurement site on a subject is determined at the PPG sensing unit 110, which may comprise at least one of: a nasal alar PPG sensor, a nasal columella PPG sensor, an ear concha PPG sensor, and a forehead PPG sensor.

In some embodiments, the method may further comprise, prior to block 202, receiving a user input at the processing unit 130 indicating at least one sensor to activate for determining the one or more PPG signals. Alternatively or in addition, the method may comprise receiving, at the processing unit 130, signal quality indicator(s) corresponding to one or more of the sensors, and one or more sensors may be activated based on the received signal quality indicator(s). Alternatively or in addition, one or more PPG sensors may be activated based on the received user input and the received signal quality indicator(s). Therefore, user(s) can decide which sensor(s) to use based on, for example, best compatibility with the user's workflow. For example, this decision may be based on whether ventilation of the subject is performed using a mask or via intubation.

Returning to Fig. 2, at block 204, a signal corresponding to chest compressions during compression therapy on the subject is received at the communication unit 120 from at least one of an external device and a motion sensing unit. The external device is one of: an external patient monitor, a defibrillator, and a CPR robot. In some embodiments, the signal received from the external device may be associated with at least one of: an impedance measurement value, a compression depth measurement value, a compression velocity measurement value, a compression acceleration measurement value, a compression force measurement value, and a robot compression signal (from a CPR robot).

Subsequently, at block 206, presence or absence of a spontaneous pulse is determined, at the processing unit 130, based on the received signal corresponding to chest compressions during compression therapy on the subject and the one or more PPG signals. This determination may be performed according to one or more algorithms developed for the purpose of detecting a return (or continued presence) of a spontaneous pulse during the application of CPR, such as the technique described in international patent application WO 2017 /211814, which includes a method that results in the output of a result of a classified pulse condition ("no pulse, "pulse", and "indeterminate"), the detection being based on compressions reference signals and PPG signals.

In some embodiments, the method may further comprise acquiring an electrocardiography (ECG) signal associated with the subject. In these embodiments, determining presence or absence of a spontaneous pulse at block 206 may further be based on the acquired ECG signal, since organized electrical activity of the heart of the subject is a prerequisite for pulse presence. The ECG signal may be acquired from an ECG sensing unit of the system, or an ECG sensing unit external to the system (e.g. an ECG sensing unit of the patient monitor).

Then, at block 208, an instruction is sent by the communication unit 120 to the external device or a user interface of the system 100 under the control of the processing unit 130, based on the presence or absence of a spontaneous pulse determined at block 206. The instruction may indicate at least one of: to output, via a user interface of the external device or a user interface of the system 100, the determined presence or absence of a spontaneous pulse , advice on further checking for pulse presence and/or withholding vasopressors to the subject, to stop compression therapy on the subject or to provide a prompt for stopping compression therapy, and to adjust a compression rate of compression therapy on the subject or to provide a prompt for adjusting the compression rate of compression therapy on the subject. In some embodiments, the instruction indicating to stop compression therapy on the subject may comprise an instruction to stop compression therapy on a regular basis for a predetermined time interval, when absence of a spontaneous pulse is determined at block 206 during compression therapy on the subject. This may allow time for determining presence/absence of a spontaneous pulse in one or more compression-free PPG signals, as compared to longer pauses of unpredictable duration in case of manual CPR. This may in turn allow more reliable pulse detection, while still maintaining a high ratio of compression to compression free time durations. Also, in some embodiments, the instruction to stop compression therapy may cause the external device to perform an automatic termination of the compression therapy upon receiving such instruction.

In some embodiments, the instruction to adjust a compression rate of compression therapy or to provide a prompt for adjusting the compression rate may be based on medical guidelines. This may allow a spontaneous pulse rate close to the compression rate to be tracked more easily.

As an example of the operation at block 208, if it is determined at block 206 that a spontaneous pulse is absent, the instruction sent to the external device or a user interface of the system 100 at block 208 may be to continue compression therapy on the subject. As another example, the instruction may indicate to output, via a display screen of the external device or a user interface of the system 100, text information which reads "increase/decrease the compression rate of compression therapy" which would prompt a user to either adjust the compression rate manually or provide a user input to the external device to adjust the compression rate of the compression therapy provided by the external device.

Although not illustrated in Fig. 2, in some embodiments the method may further comprise receiving a synchronization signal from the external device. In these embodiments, the method may further comprise performing, at the processing unit 130, synchronization of the signal corresponding to chest compressions with the one or more PPG signals based on the synchronization signal, and the step of determining presence or absence of a spontaneous pulse at block 206 may be further based on the synchronized signals.

Although not illustrated in Fig. 2, the method may further comprise determining, at the processing unit 130, whether there is an uncertain and/or weak spontaneous pulse based on the received signal corresponding to chest compressions during compression therapy on the subject and the one or more PPG signals. In these embodiments, if it is determined by the processing unit 130 that an instruction indicating to stop compression therapy on the subject is to be sent, the instruction may further comprise an instruction to stop compression therapy on a regular basis for a predetermined time interval, when it is determined that there is an uncertain and/or weak spontaneous pulse during compression therapy on the subject.

In some embodiments, the method may further comprise acquiring, at the processing unit 130, a core body temperature (CBT) value of the subject. In these embodiments, the method may further comprise controlling, at the processing unit 130, the communication unit 120 to send an instruction to the external device or a user interface of the system 100 indicating to output, via a user interface of the external device or a user interface of the system 100, at least one of: the detected core body temperature, an indication of whether the detected core body temperature is within a predetermined target range or at a predetermined target value, and an indication of whether the detected core body temperature is approaching a predetermined target range or a predetermined target value. The CBT value may be acquired from a CBT sensing unit of the system 100, and/or from a CBT sensing unit that is external to the system 100. Since during CPR a controlled decrease and/or maintenance of appropriate CBT is important to the outcome of CPR (e.g. so as to preserve brain function), by indicating the CBT (and/or whether it is within a target range or at a target value) at the external device, user(s) can be provided with useful information during CPR which can help achieve a desired outcome more efficiently and effectively.

In some embodiments, the method may further comprise acquiring, at the processing unit 130, a signal indicating nasal flow of the subject. In these embodiments, the step of controlling the communication unit to send an instruction to the external device or a user interface of the system 100 may be further based on the acquired signal indicating nasal flow of the subject. For example, an instruction (e.g. such as whether and/or when to start or restart compression therapy by a CPR robot, or follow-up action(s) after return of spontaneous circulation (ROSC)) can be provided based on a respiration rate of the subject that is derived, at the processing unit 130 or otherwise, from the signal indicating nasal flow of the subject.

The signal indicating nasal flow of the subject may be acquired from a nasal flow sensing unit of the system 100, or a nasal flow sensing unit external to the system (e.g. a nasal flow sensing unit of the patient monitor).

In some embodiments, the method may further comprise determining, at the processing unit 130, whether amplitudes and/or a signal quality corresponding to motions correlated to chest compressions during compression therapy on the subject (which may be extracted from signal(s) corresponding to chest compressions) are within a predetermined target range, and controlling, at the processing unit 130, the communication unit 120 unit to send an instruction to output, via a user interface of the external device or a user interface of the system 100, at least one of: an indication of the result of the determination of whether the amplitudes and/or the signal quality are within a predetermined target range, and an instruction to improve the amplitude and/or signal quality (e.g. to adjust a position of the motion sensing unit in a way such that sufficient and/or accurate motion sensing corresponding to compression-induced movements can be achieved).

There is thus provided an improved system and method for operating the same, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for providing cardiopulmonary resuscitation, CPR, decision support, the system comprising:
a photoplethysmography, PPG, sensing unit configured to determine one or more PPG signals at a measurement site on a subject;
a communication unit configured to receive, from at least one of an external device and a motion sensing unit, a signal corresponding to chest compressions during compression therapy on the subject, wherein the external device is one of: an external patient monitor, a defibrillator, and a CPR robot;
a processing unit configured to:
determine presence or absence of a spontaneous pulse based on the received signal corresponding to chest compressions during compression therapy on the subject and the one or more PPG signals; and
control the communication unit to send an instruction to the external device or a user interface of the system, based on the determined presence or absence of a spontaneous pulse, wherein the instruction indicates at least one of:
to output, via a user interface of the external device or a user interface of the system, at least one of: the determined presence or absence of a spontaneous pulse; and
advice on further checking for pulse presence and/or withholding vasopressors to the subject; and
to stop compression therapy on the subject or to provide a prompt for stopping compression therapy; and
to adjust a compression rate of compression therapy on the subject or to provide a prompt for adjusting the compression rate of compression therapy.

2. The system according to claim 1, wherein the communication unit is configured to be connected to the external device wirelessly or via a wired connection.

3. The system according to any one of the preceding claims, wherein the signal received from the external device is associated with at least one of: an impedance measurement value, a compression depth measurement value, a compression velocity measurement value, a compression acceleration measurement value, a compression force measurement value, and a robot compression signal.

4. The system according to any one of the preceding claims, wherein the communication unit is further configured to receive a synchronization signal from the external device, and the processing unit is further configured to perform synchronization of the signal corresponding to chest compressions with the one or more PPG signals based on the synchronization signal, and to determine presence or absence of a spontaneous pulse based on the synchronized signals.

5. The system according to any one of the preceding claims, wherein the instruction indicating to stop compression therapy on the subject comprises an instruction to stop compression therapy on a regular basis for a predetermined time interval, when absence of a spontaneous pulse is determined during compression therapy on the subject.

6. The system according to any one of the preceding claims, wherein the processing unit is further configured to determine whether there is an uncertain and/or weak spontaneous pulse based on the received signal corresponding to chest compressions during compression therapy and the one or more PPG signals, and
wherein the instruction indicating to stop compression therapy on the subject comprises an instruction to stop compression therapy on a regular basis for a predetermined time interval, when it is determined that there is an uncertain and/or weak spontaneous pulse during compression therapy on the subject.

7. The system according to any one of the preceding claims, wherein the PPG sensing unit comprises at least one of: a nasal alar PPG sensor, a nasal columella PPG sensor, an ear concha PPG sensor, and a forehead PPG sensor.

8. The system according to claim 7, wherein the PPG sensing unit comprises more than one of: a nasal alar PPG sensor, a nasal columella PPG sensor, an ear concha PPG sensor, and a forehead PPG sensor, and the processing unit is configured to receive a user input indicating at least one sensor to activate for determining the one or more PPG signals.

9. The system according to claim 7 or claim 8, wherein the PPG sensing unit comprises more than one of: a nasal alar PPG sensor, a nasal columella PPG sensor, an ear concha PPG sensor, and a forehead PPG sensor, and wherein the processing unit is configured to:
receive one or more signal quality indicators corresponding to one or more of the PPG sensors; and
determine at least one of the one or more PPG sensors to activate based on the received one or more signal quality indicators.

10. The system according to any one of the preceding claims, wherein the processing unit is further configured to:
acquire a core body temperature value of the subject; and
control the communication unit to send an instruction indicating to output, via a user interface of the external device or a user interface of the system, at least one of: the detected core body temperature, an indication of whether the detected core body temperature is within a predetermined target range or at a predetermined target value, and an indication of whether the detected core body temperature is approaching a predetermined target range or a predetermined target value.

11. The system according to any one of the preceding claims, wherein the processing unit is further configured to acquire an electrocardiography, ECG, signal associated with the subject, and wherein determining presence or absence of a spontaneous pulse is further based on the acquired ECG signal.

12. The system according to any one of the preceding claims, wherein the processing unit is further configured to acquire a signal indicating nasal flow of the subject, wherein controlling the communication unit to send an instruction to the external device or a user interface of the system is further based on the acquired signal indicating nasal flow of the subject.

13. The system according to any one of the preceding claims, wherein the processing unit is further configured to:
determine whether amplitudes and/or a signal quality corresponding to the detected motions correlated to chest compressions during compression therapy on the subject are within a predetermined target range; and
control the communication unit to send an instruction to output, via a user interface of the external device or a user interface of the system, at least one of: an indication of the result of the determination of whether the amplitudes and/or the signal quality are within a predetermined target range, and an instruction to improve the amplitude and/or signal quality.

14. The system according to any one of the preceding claims, wherein the processing unit is implemented as part of the external device, and the processing unit is configured to connect to the communication unit and the PPG sensing unit wirelessly.

15. A method for operating a system for providing cardiopulmonary resuscitation, CPR, decision support, wherein the system comprises a photoplethysmography, PPG, sensing unit, a communication unit, and a processing unit, the method comprising:
determining, at the PPG sensing unit, one or more PPG signals at a measurement site on a subject;
receiving, at the communication unit, a signal corresponding to chest compressions during compression therapy on the subject from at least one of an external device and a motion sensing unit, wherein the external device is one of: an external patient monitor, a defibrillator, and a CPR robot;
determining, at the processing unit, presence or absence of a spontaneous pulse based on the received signal corresponding to chest compressions during compression therapy on the subject and the one or more PPG signals; and
controlling, at the processing unit, the communication unit to send an instruction to the external device or a user interface of the system, based on the determined presence or absence of a spontaneous pulse, wherein the instruction indicates at least one of:
to output, via a user interface of the external device or a user interface of the system, at least one of the determined presence or absence of a spontaneous pulse; and
advice on further checking for pulse presence and/or withholding vasopressors to the subject; and
to stop compression therapy on the subject or to provide a prompt for stopping compression therapy; and
to adjust a compression rate of compression therapy on the subject or to provide a prompt for adjusting the compression rate of compression therapy.
